# EUROPEAN PATENT SPECIFICATION

(11) **EP 3 434 686 B1**
(45) Date of publication and mention of the grant of the patent: **01.10.2025**
(21) Application number: 16894820.6
(22) Date of filing: 10.05.2016
(51) Int. Cl.: C07K 14/59, C12N 15/16, C12N 15/66, A61K 38/24, G01N 33/74, G01N 33/76, A61P 15/08

(54) **METHOD FOR PRODUCING AND PURIFYING HYBRID OR NON-HYBRID RECOMBINANT GLYCOPROTEIN HORMONES, HYBRID OR NON-HYBRID RECOMBINANT GLYCOPROTEIN HORMONES, EXPRESSION VECTORS AND USES OF THE RECOMBINANT GLYCOPROTEIN HORMONES**
VERFAHREN ZUR HERSTELLUNG UND REINIGUNG VON HYBRIDEN ODER NICHT-HYBRIDEN REKOMBINANTEN GLYKOPROTEINHORMONEN, HYBRIDE ODER NICHT-HYBRIDE REKOMBINANTE GLYKOPROTEINHORMONE, EXPRESSIONSVEKTOREN UND VERWENDUNGEN DER REKOMBINANTEN GLYKOPROTEINHORMONE
PROCÉDÉ DE PRODUCTION ET DE PURIFICATION D'HORMONES GLYCOPROTÉIQUES RECOMBINANTES HYBRIDES OU NON HYBRIDES, HORMONES GLYCOPROTÉIQUES RECOMBINANTES HYBRIDES OU NON HYNRIDES, VECTEURS D'EXPRESSION ET UTILISATIONS DE CES HORMONES GLYCOPROTÉIQUES RECOMBINANTES

(30) Priority: 22.03.2016 BR 10201606222
(43) Date of publication of application: 30.01.2019
(73) Proprietor: Universidade de São Paulo - USP, 05508-220 São Paulo (BR); LCR Kimera Biotecnologia Ltda - ME, 14056-680 Ribeirão Preto - SP (BR)
(72) Inventor: BARUFFI, Marcelo Dias, 14050-470 Ribeirão Preto - SP (BR); ANDRADE, Camillo del Cistia, 14110-000 Ribeirão Preto - SP (BR); SILVA, Rubens Eduardo da, 14031-868 Ribeirão Preto - SP (BR); OLIVEIRA, Robinson Antonio Martins de, 14026-090 Ribeirão Preto - SP (BR); CALLEJON, Daniel Roberto, 14026-587 Ribeirão Preto - SP (BR)
(74) Representative: Clarke, Modet y Cía., S.L.
(86) International application number: PCT/BR2016/000051
(87) International publication number: WO 2017/161427

(56) References cited:
- EP-A1- 0 974 599
- WO-A1-2012/131306
- WO-A1-2015/124945
- WO-A1-90/02812
- WO-A1-96/05224
- SÉBASTIEN LEGARDINIER ET AL: "Stability and biological activities of heterodimeric and single-chain equine LH/chorionic gonadotropin variants", JOURNAL OF MOLECULAR ENDOCRINOLOGY., vol. 40, no. 4, 15 February 2008 (2008-02-15), GB, pages 185 - 198, XP055557280, ISSN: 0952-5041, DOI: 10.1677/JME-07-0151
- DATABASE DATABASE [o] 22 May 1998 (1998-05-22), "Equine chorionic gonadotropin alpha and beta chain fusion protein", Database accession no. WO 9821238 A1
- YOUNG, C. L. ET AL.: "Recombinant protein expression and purification: a comprehensive review of affinity tags and microbial applications", BIOTECHNOL J., vol. 7, no. 5, 7 May 2012 (2012-05-07), pages 620 - 634, XP055104740
- GARCIA-CAMPAYO, V. ET AL.: "Novel recombinant gonadotropins", TRENDS IN ENDOCRINOLOGY & METABOLISM ., vol. 12, no. 2, March 2001 (2001-03-01), pages 72 - 77, XP001071097, ISSN: 1043-2760, Retrieved from the Internet <URL:https://doi.org/10.1016/S1043-2760(00)00338-6>

## Description

The present invention belongs to the field of processes for producing peptide hormones; specifically, it belongs to the field of processes for producing peptide hormones containing more than 20 amino acids; and describes a process for producing and purifying hybrid or non-hybrid recombinant glycoprotein hormones, hybrid or non-hybrid recombinant glycoprotein hormones, including their expression vectors, and uses thereof.

### BACKGROUND OF THE INVENTION

In recent years, numerous biotechnological processes of production and purification of protein and glycoprotein hormones have been developed. All processes developed until then have their own strategies that vary according to the hormone to be produced and that aim at increasing the production of the hormone, or are aimed at facilitating the purification step.

In the production of recombinant glycoproteins, the state of the art uses mammalian cells due to their ability to promote the correct folding and post-translational processing. Several factors are involved in the optimization of protein expression in mammalian cells. One of these factors is the expression vectors for generation of recombinant cell lines using strong promoters of viral or cellular origin, such as the cytomegalovirus (CMV) promoter (Gopalkrishnan et al., 1999). Currently, most of the high protein production processes for the Biopharmaceutical industry (about 60-70%) are based on cells grown in suspension (Moritz, et al, 2015).

Equine Chorionic Gonadotropin (eCG) is a glycoprotein hormone produced in the trophoblast of pregnant mares, consisting of 2 subunits (α and β), with similar action on the follicle stimulating hormone (FSH) and luteinizing hormone (LH), both from the hypophysis and with important action in the events of induction of follicular growth and luteinization, respectively (Murphy, 2012). This bi-functional hormonal activity occurs after the administration of eCG in species of mammals other than horses, such as cattle, swine, sheep and goats (Murphy, 2012). The N-glycosylation sites of the alpha chain of the eCG is fundamental for the expression of its LH activity (Min et al. 1996; Min et al., 2004; Bousfield et al., 2004; Murphy, 2012). The loop region of the eCG protein structure and a sequence of amino acid residues (104-109) of the C-terminal region of the beta chain of this hormone are associated with the bi-functional action of eCG (LH and FSH activity) and its FSH function, respectively (Moyle et al. 1994; Galet et al. 2009).

eCG is used in different protocols of assisted animal reproduction. The use of eCG in other mammals may induce the production of anti-eCG antibodies and they may decrease the biological actions of this hormone in these animals (Hervé et al. 2004, Forcada et al. 2011). The alpha chain of the eCG molecule is the major antigenic portion of this hormone (Chopineau et al, 1993).

The eCG gene is present on chromosome 10 in *Equus caballus,* and its expression generates the subunits, (i) gonadotropin alpha 1 subunit (chr10: 39937900-39940069; Gene ID: 100034174), its transcription undergoes splicing of 3 exons generating a messenger (mRNA) of nearly 2 Kb and an open reading frame (ORF) of 363 nucleotides translated into a mature protein of 120 amino acids and approximately 13.8 kDa, and (ii) chorionogonadotropin subunit beta (chr10: 18963366-18964444; Gene ID: 100054774), its transcription undergoes splicing of 3 exons generating a mRNA of approximately 520 kb and its open reading frame (ORF) of 510 nucleotides is translated into a mature protein of 169 amino acids and approximately 17.8 kDa (http://genome.ucsc.edu, http://www.ncbi.nlm.nih.gov).

The CGA gene of the Bos taurus species is present on chromosome 9 at position chr9: 63692501-63694585, and its expression generates the subunit, gonadotropin alpha 1 subunit (Gene ID: 280749), its transcription undergoes splicing of 4 exons generating a mRNA of approximately 742 base pairs (bp) (provisional to date) and open reading frame (ORF) of 363 nucleotides translated into a mature protein of 120 amino acids and approximately 13.6 kDa (http://genome.ucsc.edu, http://www.ncbi.nlm.nih.gov).

The CGA gene of the Sus scrofa species is present on chromosome 10 at position chr10: 62246069-62248001, and its expression generates the subunit, gonadotropin alpha 1 subunit (Gene ID: 406869), its transcription undergoes splicing of 3 exons generating a mRNA of approximately 363 base pairs (bp) (provisional to date) and open reading frame (ORF) of 363 nucleotides translated into a mature protein of 120 amino acids and approximately 13.5 kDa (http://genome.ucsc.edu, http://www.ncbi.nlm.nih.gov).

The CGA gene of the Ovis aries species is present on chromosome 8 at position chr8: 49919904-49921988, and its expression generates the subunit, gonadotropin alpha 1 subunit (Gene ID: 443538), its transcription undergoes splicing of 4 exons generating a mRNA of approximately 716 base pairs (bp) (provisional to date) and open reading frame (ORF) of 363 nucleotides translated into a mature protein of 120 amino acids and approximately 13.5 kDa (http://genome.ucsc.edu, http://www.ncbi.nlm.nih.gov).

The CGA gene of the Capra hircus species is present on chromosome 8 at position chr8: 49919901 -49921988, and its expression generates the subunit, gonadotropin alpha 1 subunit (Gene ID: 100860817), its transcription undergoes splicing of 3 exons generating a mRNA of approximately 366 base pairs (bp) (provisional to date) and open reading frame (ORF) of 363 nucleotides translated into a mature protein of 120 amino acids and approximately 13.5 kDa (http://genome.ucsc.edu, http://www.ncbi.nlm.nih.gov).

### STATE OF THE ART

Several patent documents relate to processes of producing chorionic gonadotrophin from equine and other mammals. For example:
Document EP0974599 discloses a recombinant equine chorionic gonadotropin hormone in which the α and β chains of equine chorionic gonadotropin are bonded. This patent also claims the veterinary uses of this recombinant molecule.

On the other hand, document PI 0108556-5 describes a purification process of recombinant human Chorionic Gonadotropin (rhCG) produced in CHO cell cultures, which comprises the combined use of ion exchange chromatography and reverse phase HPLC. Such document also claims a pharmaceutical composition containing rhCG for subcutaneous administration.

Documents PI 9814880-0 and PI 9914670-3 relate to single-chain recombinant glycoprotein hormones, the process of producing the same without the use of a purification system by affinity chromatography, of fluorescent label and a polypeptide characteristic of cell secretion.

Document US5260421 discloses a method of promoting site-directed mutagenesis in glycoproteins in general, for the production of hormones, such as luteinizing hormone, follicle stimulating hormone, thyroid stimulating hormone, and chorionic gonadotrophin. Document US6469139 discloses a modified human chorionic gonadotrophin at specific sites of the amino acid sequence, and its medical use as an immunological contraceptive.

Document WO9532216 discloses a method of producing biologically active glycoprotein hormones in prokaryotic cells which employs a redox thiol buffer to form structurally active subunits of the hormone.

Documents JPH1036398 and JPH1036399 relate to processes of producing recombinant equine chorionic gonadotropin, in which the subunits are not fused in a single-chain. The difference between them is due to the fact that the former claims the use of r-eCG in Al procedures or superovulation in cattle, while the latter claims the activity of stimulating the production of FSH.

Document WO2014183175 discloses methods for the production and purification of follicle stimulating hormone (FSH) using a parent or mutant HEK 293 cell platform.

Thus, no reports were found in the state of the art concerning the methods of obtaining and using artificial insemination and superovulation protocols related to the hybrid forms of chorionic gonadotrophin composed by the association of non-equine alpha chains and equine beta chains.

Database 22 May 1998 "Equine chorionic gonadotropin alpha and beta chain fusion protein" discloses the fusion protein of the beta and alpha-subunits of equine CG.

WO 96/05224 A1 discloses single-chain forms of the glycoprotein hormone quartet. The document refers to single-chain forms, wherein the α and β subunits of the wild-type heterodimers or their variants are covalently linked, optionally through a linker moiety; and also single-chain compounds comprising two β subunits of the glycoprotein hormones, which β subunits are the same or different. These 'two-β' forms are antagonists of glycoprotein hormone activity.

### SUMMARY OF THE INVENTION

The present invention aims to propose a process for producing and purifying hybrid recombinant glycoprotein hormones.

In addition, the present invention proposes recombinant glycoprotein hormones of equine origin (r-eCG) and other hybrids containing portions of equine (β chain) and target mammalian origin (α chain), resulting in chimeric glycoprotein hormones specific to the target species, aiming at obtaining a hormonal composition that possesses the LH and FSH activities and without immunotoxicity to the target species.

In addition, the present invention further proposes the hybrid recombinant glycoprotein hormones obtained with the use of their expression vectors and their pharmaceutical compositions, for use in assisted animal reproduction of target species of commercial or non-commercial interest, mammals in general, like cattle, sheep, goats, pigs, horses, mules, bubainos, bison, antelopes, domestic and wild species of canines and felines, cetaceans, ursids and primates.

### BRIEF DESCRIPTION OF DRAWINGS

Figure 1 shows the electrophoretic analysis of the amplification procedure of the gene fragment referring to SEQ. ID. 16;
Figure 2 shows the electrophoretic analysis of cleavage products of recombinant clones (SEQ. ID. 16) of SEQ. ID. 38;
Figure 3 shows the electrophoretic analysis of cleavage products of recombinant clones (SEQ. ID. 16) of SEQ. ID. 39;
Figure 4 shows the expression analysis of expression of the GFP molecule in CHO-K1 cells transfected with SEQ. ID. 39, by fluorescence microscopy, where (A) and (C) illustrate DIC (Differential Interference Contrast) images and show a similar cell growth pattern between the two cell populations, while (B) and (D) illustrate the fluorescence related to the presence of the GFP protein;
Figure 5 shows the electrophoretic analysis of cell culture supernatant containing the SEQ. ID. 17 (non-fused recombinant r-eGG to GFP molecule); in which it is possible to observe in MM - Molecular Marker; MC - Culture medium (Freestyle Serum Free, C - HEK 293 cells after 48 hour culture; SB - Supernatant of HEK 293 cell culture after 48 hour culture, where it is possible to observe the SEQ. ID. 17 band;
Figure 6 shows the electrophoretic analysis of purified preparation of SEQ. ID. 19. 12% SDS-PAGE, where it is possible to observe in MM - Molecular Marker; C - Culture Medium (DMEM containing 10% fetal bovine serum); S+ - Cell culture supernatant; FT - Flow Through, proteins that did not bind to the His-Trap column; E1 to 5 - Fractions eluted from the column, where it is possible to observe the band referring to the SEQ. ID. 19;
Figure 7 shows purified preparations of SEQ. ID. 19 inducing the release of estradiol and progesterone in the serum of rats;
Figure 8 shows the analysis of in vivo activity of SEQ. ID. 17 corresponding to non-fused recombinant eCG to GFP and purified from culture of the supernatant of HEK 293 cells cultured in the absence of fetal bovine serum using the Freestyle Serum Free culture medium;
Figure 9 shows the functional comparative analysis between the recombinant forms of the control molecule (SEQ. ID: 49), of native eCG and SEQ. ID. 19, where (A) represents images of the ovaries of intramuscularly treated prepubertal rats with PBS (negative control), SEQ. ID. 49, native eCG and SEQ. ID. 19;

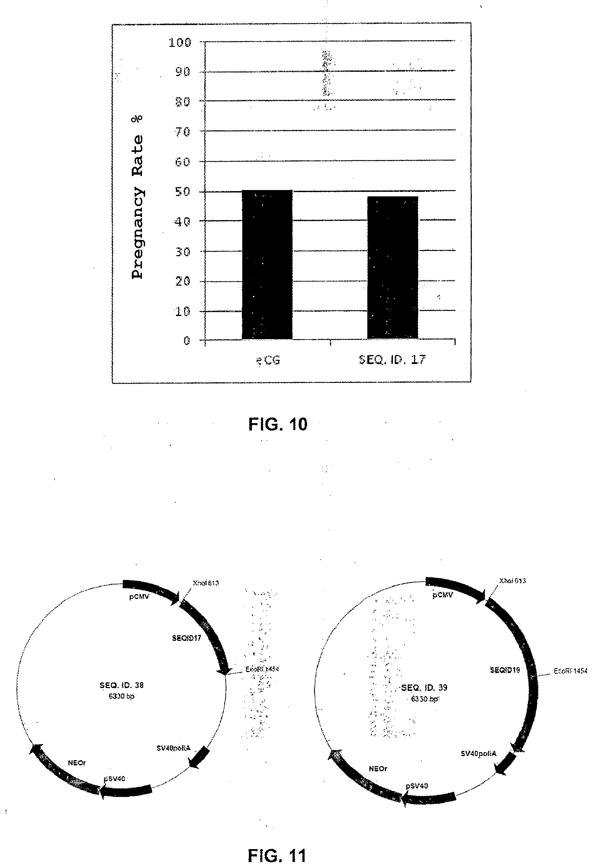

Figure 10 shows the comparative analysis of the pregnancy rate in females, for the evaluation of the activity of SEQ. ID. 17 in large animals (cattle). Graphic representation of the percentage of pregnancy rate of females induced to estrus through hormonal protocols performed by the administration of eCG 300 IU and of SEQ. ID. 17 30 µg. The analysis was performed by ultrasonography after 30 days of insemination of Bos taurus indicus females, with homogeneous groups for the animal category (race, age, calving at least 1 time), with n = 127 for the eCG group and n = 50 for the group SEQ. ID. 17 group.
Figure 11 shows the graphic representation of vectors relating to SEQ. ID. 38 and SEQ. ID. 39, which represent all the vectors described in this invention.

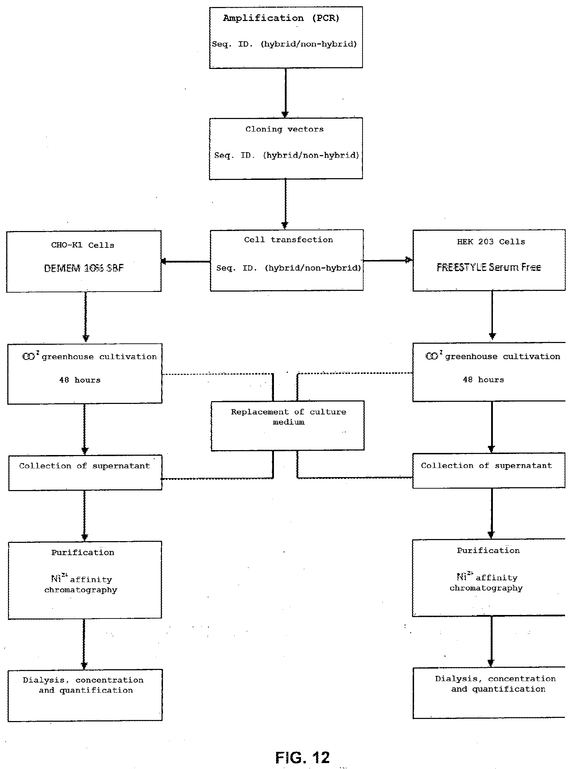

Figure 12 shows the organization chart of the steps of the production and purification process of recombinant glycoprotein hormones of this invention.

### DETAILED DESCRIPTION OF THE INVENTION

The present invention relates to a process for the production and purification of hybrid chorionic gonadotropin comprising the steps of:
(a) amplification and cloning of a DNA molecule encoding a polypeptide selected from the group consisting of SEQ ID NO:23, SEQ ID NO: 25, SEQ ID NO: 27, SEQ ID NO: 29, SEQ ID NO:31, SEQ ID NO:33, SEQ ID NO:35 and SEQ ID NO:37 and the use of nucleotide sequences of primers of the different forms of chorionic gonadotrophin and of cleavage sites for restriction enzymes and of DNA sequences coding for a histidine tail and a proteolytic site for TEV-Tag protease;
(b) construction of the expression vectors comprising the sequences of step (a);
(c) transfection of a mammalian cell line with the vector of step (b), expression and analysis of cells;
(d) purification of the hybrid chorionic gonadotropin by affinity chromatography, wherein the hybrid chorionic gonadotropin is selected from the group consisting of SEQ ID NO: 23, SEQ ID NO: 25, SEQ ID NO: 27, SEQ ID NO: 29, SEQ ID NO: 31, SEQ ID NO: 33, SEQ ID NO: 35 and SEQ ID NO: 37;
(e) dialysis and sterilization of the hybrid chorionic gonadotropin obtained in step (d).

Hybrid glycoprotein hormone (r-eCG), whether or not fused to the GFP molecule, of the present invention, are selected from the group consisting of recombinant equine chorionic gonadotrophin (r-eCG), recombinant bovine chorionic gonadotrophin (r-bCG), recombinant suine chorionic gonadotrophin (r-sCG), recombinant ovary chorionic gonadotrophin (r-oCG), recombinant goat chorionic gonadotrophin (r-cCG), recombinant thyroid stimulating hormone (r-TSH), recombinant luteinizing hormone (r- and recombinant follicle stimulating hormone (r-FSH). Preferably, the hybrid hormone eCG, fused or not to the GFP molecule, represents, respectively, the nucleotide and glycoprotein corresponding to the hybrid equine chorionic gonadotrophin (r-eCG).
(a) amplification, modification and cloning of the hybrid molecules;

The step of PCR amplification of the r-eCG gene fragments (SEQ.ID. 16) or r-eCG-GFP (SEQ.ID. 18) by PCR (Mullis et al., 1986) comprises the use of primer oligonucleotides of SEQ. ID. 1, SEQ. ID. 2 and SEQ. ID. 3 complementary to the different forms of native chorionic gonadotrophin to obtain a gene fragment relating to the fusion between the beta subunit DNA sequence of the native eCG (SEQ. ID. 6) and the native eCG alpha subunit DNA sequence (SEQ. ID. 4), wherein the SEQ. ID. 6 and 4 correspond to the α and β subunits of eCG and additional sequences corresponding to their total in SEQ ID. 16 or SEQ. ID. 18 validated by agarose gel electrophoresis.

SEQ ID. 1, SEQ. ID. 2 and SEQ. ID. 3, in addition to promoting the amplification of the genes related to eCG subunits α and β subunits, present additional nucleotide sequences associated with restriction enzyme cleavage sites and coding sequences for a histidine tail for poly-histidine sequence translation (6 x His-Tag), and a proteolytic site, such as the Tobacco Etch Virus (TEV-Tag) protease site, associated with cloning of the gene sequences, purification of recombinant hormones and protein editing of these hormones respectively, thereby generating a fragment of DNA with 847 bp.

Amplification occurs by PCR, the polynucleotide of SEQ. ID. 16 being obtained, which is then purified on chelating resin (Sambrook et al, 1989).
(b) Construction of vectors for the expression of hybrid chorionic gonadotropin in eukaryotic cells (CHO-K1 and HEK 293)

The construction of vectors for the expression of the hybrid chorionic gonadotropin in eukaryotic cells (CHO-K1 and HEK 293) is initiated by the cloning of SEQ. ID. 16 or SEQ. ID. 18 in prokaryotic cells (E. coli DH5a). This cloning step begins with the insertion of the sequences SEQ. ID. 16 or SEQ. ID. 18 in a commercial cloning vector that is used to transform DH5α competent cells by thermal shock. Finally, the selection of bacterial recombinant clones containing the recombined cloning vector is performed with the sequences SEQ. ID. 16 or SEQ. ID. 18, whose presences are validated by agarose gel electrophoresis and DNA chemical sequencing. It should be clear for a person skilled in the art that various techniques and reagents may be used without the difference between the techniques and reagents being able to generate significant differences in the final process. In the present invention, the transformation of competent E. coli DH5α prokaryotes is accomplished by the introduction of cloning vectors by thermal shock, and the selection of recombinant clones is performed by cleavage for the detection of SEQ. ID. 16 and confirmation of the sequence by a chemical method of nucleotide sequencing, described in the literature (Sanger et al, 1997).
(c) Transfection, expression and analysis of cells that produce hybrid glycoprotein hormones.

Expression vectors obtained after the cloning step of the hybrid hormones are then used to transiently transfect eukaryotic cells with the aid of liposomes. Alternatively, the recombinant hormone gene sequences used in the composition of the expression vectors for unstable eukaryotic cell transformation may be used in the construction of expression vectors for stable transformation of these cells with the use of sequences. SEQ. ID. 16 or SEQ. ID. 18, via lentiviral vectors or biologically safe systems, of non-random gene integration and without the need for selective agents (antibiotics and other chemical substances) such as Transcription Activator-Like Effector Nucleases (TALENs), Clustered Regularly Interspaced Short Palindromic Repeats (CRISPR) and other systems with these properties, for the generation of expression cell lines and their analysis by fluorescence microscopy for visualization of the protein expression of SEQ. ID. 19 and/or by other methodologies (immunodetection or gene sequencing) for detecting expression of the protein of SEQ. ID. 17 and SEQ. ID. 19. On the other hand, stable integration systems such as the use of lentiviral vectors can be used for these same purposes.

It is possible to detect, in the fluorescence microscope analysis, the change in fluorescence of the cells of the culture medium, since SEQ. ID. 19 is exported to culture medium due to its signaling peptide of SEQ. ID. 21, present between amino acids 1-20 of SEQ. ID. 19. In control cells transfected with SEQ. ID. 48, the presence of fluorescence is only detectable in the cell cytoplasm.
(d) Purification of hybrid chorionic gonadotropin

Purification occurs by collecting the supernatant from the culture medium of mammalian cells transfected with the sequences SEQ. ID. 16 or SEQ. ID. 18 and that secret the SEQ. ID. 17 or SEQ. ID. 19, transiently or stably, followed by centrifugation between 1200 and 1800 g between 7 and 15 minutes at 4 °C for the removal of cells in suspension and subsequent purification of SEQ. ID 17 or SEQ. ID. 19 by affinity chromatography on nickel resins and after elution, an approximate yield of 15 to 17 mg of purified hormone is obtained for each liter of culture.
(e) Dialysis and sterilization of hybrid chorionic gonadotropin.

The final step to obtain the hybrid chorionic gonadotropin is carried out by dialysis in concentrators and/or by tangential centrifugation (cut off of 10 to 60 kDa), where the buffer is used in the production and purification steps of glycoprotein hormones in PBS pH 7.4 is changed, followed by sterilization of the solution containing the hormones produced with filters (0.22 µm) suitable for the final volume.

The invention also relates to a chorionic gonadotropin comprising the subunits α and β fused in a single chain and chain modifier agents at the amino and carboxy terminal moieties, such as a fusion site to a fluorescein, such as GFP; a purification marker, such as a poly-His tail; peptide signaling the secretion of the molecule; dimerization interface peptide and a specific proteolytic site, such as the proteolytic cleavage site with the TEV (Tobacco etch virus) proteolytic enzyme, and optionally, a fluorescent label. These modifications provide the recombinant molecule with features not presented by wild-type hormones, such as nickel affinity, alpha- and beta-strand fusion and / or fluorescence emission, which favor its production and purification processes.

All cloning, expression and purification steps described in steps (a) to (d) related the preparation of r-eCG were efficient and effective and should also be used in the cloning, expression and purification steps of the hybrid forms of chorionic gonadotropin.

Preferably, this production process is used for the production of SEQ. ID. 17 and SEQ. ID. 19, referring to hybrid equine corionic gonadotrophin which, in vivo, had a bioactivity of approximately 10,000 IU/mg and close to the bioactivity of native eCG preparations.

Aiming at a reduction of the immunogenicity of SEQ. ID. 17 and SEQ. ID. 19 for other species, this invention aimed at obtaining hybrid forms of these glycoprotein hormones composed of the alpha chain of the target species and the equine beta chain with and/or without fusion with the GFP molecule.

### Examples for obtaining functional analysis of r-eCG

### Example 1- Construction of the cloning vector of SEQ. ID. 16:

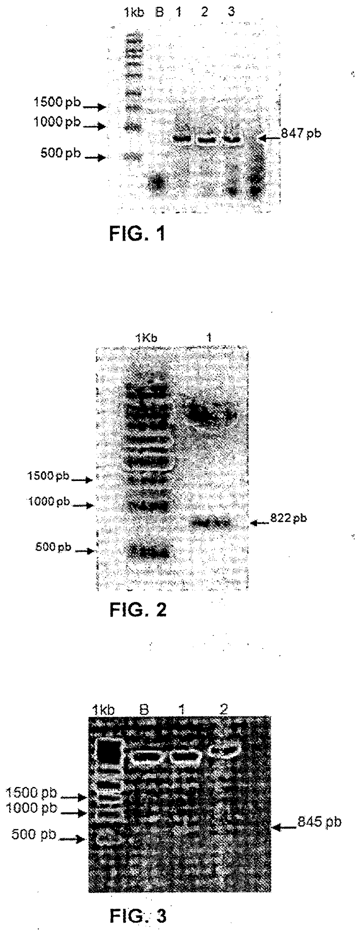

The elaborated gene fragment related to SEQ. ID. 16 was commercially synthesized and amplified by PCR (FIG. 1), using the oligonucleotides (SEQ. ID. 1, SEQ ID. 2 and SEQ. ID. 3). The electrophoretic analysis of the amplification procedure was performed on agarose gel (1%) and with the use of a 1Kb molecular size marker. The samples tested were: negative control of the PCR reaction (lane B); and gene fragment (SEQ. ID. 16) amplified of approximately 847 bp (lanes 1, 2 and 3).

SEQ. ID. 16, was cloned into a plasmid vector (CloneJet-Thermo) and used to transform W5a competent E. coli cells by heat shock, and, after selection of recombinant clones by cleavage, the sequence confirmation of SEQ. ID., 16 was performed by a chemical nucleotide sequencing method.

### Example 2 - Construction of the expression vectors of SEQ. ID. 38 and SEQ. ID. 39.

Once the SEQ. ID. 16 was confirmed, the recombinant clones were cleaved with the Xhol and EcoRI enzymes, for removing the fragment of the SEQ ID. 16, and cloned for generating SEQ. ID. 38 and SEQ. ID. 39. Selection of recombinant clones was done by cleavage of SEQ. ID. 38 and SEQ. ID. 39. The electrophoresis of clone cleavage products was done on agarose gel (1%) using a 1Kb molecular marker. As seen in figure 2, the clone after cleavage with Xhol and EcoRI is found in lane 1, where the band relative to SEQ. ID. 16 is seen. In figure 3, the samples tested were: negative control of the PCR reaction (lane B); where the clones after cleavage of SEQ. ID. 39 are in lanes 1 and 2), where the band referring to SEQ. ID. 16 is seen. Confirmation of the perfect sequence of SEQ. ID. 16 was carried out by chemical DNA sequencing.

### Example 3 - Transfection of mammalian cells:

For the generation of expression cell lines (HEK 293 and CHO-K1), 6-well 500 µL plates (24-well plates) were used for transfecting 800 ng of SEQ. ID. 38 and SEQ. ID. 39, using 2 µL of lipofectamine 2000 (Thermo). As a control, cells were transfected under the same conditions with SEQ. ID. 48. Cells were cultured on Freestyle Serurm Free (Thermo) or DMEM medium (Sigma) containing 10% fetal bovine serum and 1x antibiotic/antimycotic solution for 24 hours for further addition of 400 µg/mL geneticin (G418, Sigma-Aldrich).

### Example 4 - Selection of transfected mammalian cell clones:

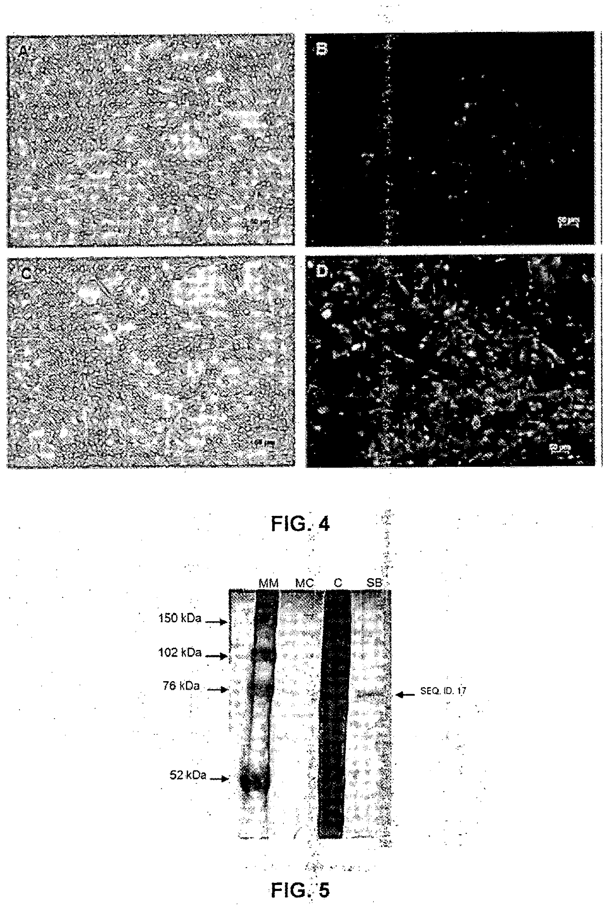

Transfected cells were selected over a period of 3 weeks, with geneticin concentration (G418) changes for elimination of non-transfected clones. Cells were then analyzed using fluorescence microscopy for the expression of SEQ. ID. 19 and of SEQ. ID. 49. The analysis was made by observing the change in the fluorescence of the cells and the culture medium, since SEQ. ID. 19 is exported to the medium due to its signal peptide present between amino acids 1-20 (SEQ. ID. 21). The control cells showed the presence of fluorescence only within the cells, due to the expression of SEQ. ID. 49 (Figure 4).

Cells transfected with SEQ. ID. 39 express and export SEQ. ID. 19 to the culture medium (figure 4 (B)). Cells transfected with SEQ. ID. 48 express the GFP protein (SEQ. ID. 49) in the cells (Figure 4 (D)).

### Example 5 - Purification and electrophoretic analysis of SEQ. ID. 17 and SEQ. ID. 19:

The selected HEK 293 cells were transferred to Spinner with 100 mL of Freestyle (Thermo) culture medium containing 400 mg/mL geneticin for propagation, increasing the number of cells in the highest culture volume and consequently the concentration of recombinant protein expressed for 96 hours.

The selected CHO-K1 cells were transferred to 75 cm² culture bottles containing DEMEN (Sigma) containing 10% fetal bovine serum and geneticin 400 mg/mL for propagation, increasing the number of cells in the highest culture volume and consequently the concentration of recombinant protein expressed for 8 days of propagation with collections of supernatant (culture medium) every 48 hours.

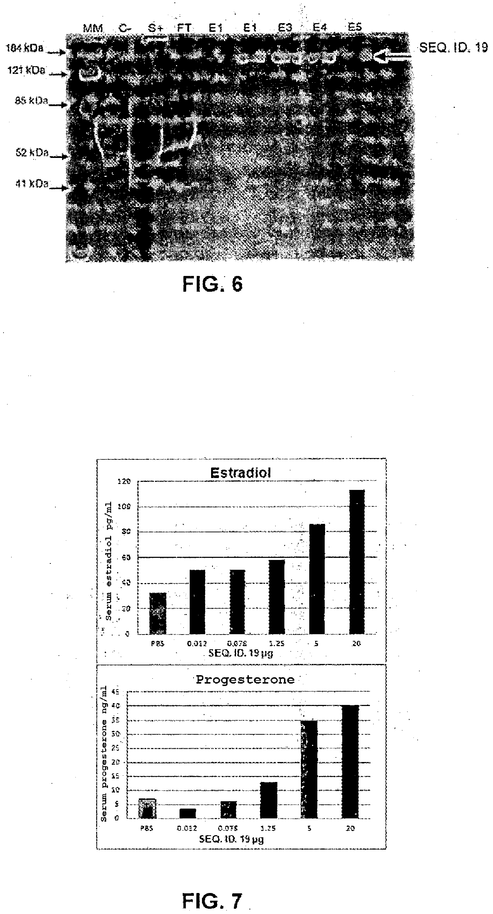

After culture of the HEK 293 and CHO-K1 cells, centrifugation (1500 x g/10 min./4 °C) of the culture media was carried out for the removal of cells in suspension, concentration and dialysis in appropriate concentrators, and for further purification of SEQ. ID. 17 and SEQ. ID. 19 by His-Trap column affinity chromatography on a suitable chromatograph. After elution with imidazole gradient (Sigma) (5 to 500 mM), the fractions were analyzed on 12% SDS-PAGE, as seen in figure 6. After concentration of the eluted fractions, quantification by absorbance measurement at 280 nm and correction by the correction factor (calculated by the molar extinction coefficient) of 1.29 for SEQ. ID. 17 e 1,34 for SEQ. ID. 19, an approximate yield of 15 mg of purified SEQ. ID. 17 and 17 mg of purified SEQ. ID. 19 for each liter of culture was estimated.

### Example 6 - Verification of the ability of SEQ. ID. 19 to induce the production of hormones in vivo.

The hormonal effects of SEQ. ID. 19 were evaluated by rat assays and quantified by the chemiluminescence technique. Figure 7 shows the induction of the production of estradiol (17β-estradiol) and progesterone measured in the serum of immature (Wistar) rats at 4-6 weeks of age after 6 and 18 hours, respectively, of the intramuscular injection of decreasing doses of SEQ. ID. 19 (0.012 to 20 µg), using as a control 4-6 week old immature rats injected with phosphate buffered saline, pH = 7.4 (PBS); the procedures used in the hormone induction experiments in rats were approved by the Ethics Committee on the Use of Animals of the Ribeirão Preto Campus of the University of Sao Paulo (CEUA) - (Protocol No. 14.1.479.53.0).

### Example 7 - Analysis of the ability of SEQ. ID. 17 and of SEQ. ID. 19 to induce increased ovarian mass in rats:

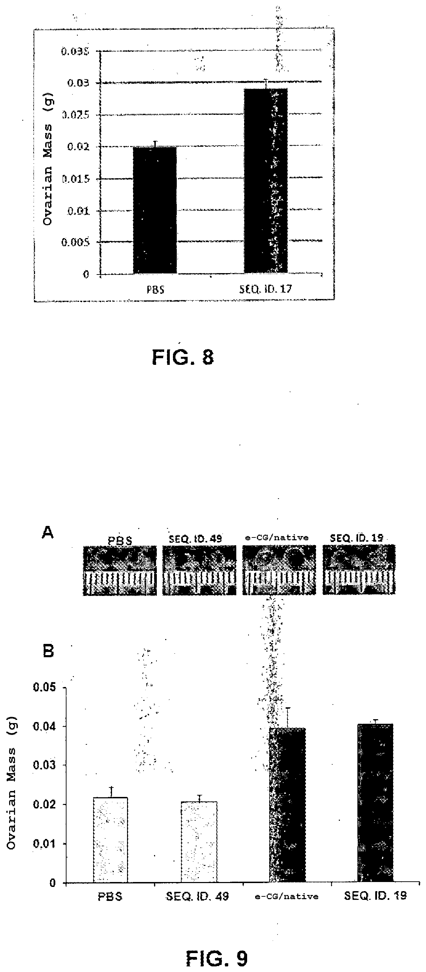

The assessment of the ability of SEQ. ID. 17 and SEQ. ID. 19 to promote activity *in vivo* related to the hormonal function of chorionic gonadotrophin was analyzed by the measurement of the ovarian mass of rats treated with SEQ. ID. 17 [r-eCG without GFP (10 up)] and SEQ. ID. 19 [r-eCG with GFP (20 ug)]. The effects of SEQ. ID. 17 on ovarian growth induction were then evaluated by measuring the ovarian mass of rats (Wistar) at 21 days after intramuscular injection of 10 ug of SEQ. ID. 17 (fig. 8). Each experimental group contained 4 animals (total of 8 ovaries per group). The procedures used in the induction experiments of ovarian mass increase in rats were approved by CEUA (Protocol No. 14.1.479.53.0).

Likewise, figure 9 shows the functional analysis (induction of ovarian mass increase) comparative between the recombinant forms of Green Fluorescent Protein (GFP, SEQ. ID. 49), the native form of eCG (SEQ. ID. 5 and SEQ. ID. 7) and of SEQ. ID. 19. Each experimental group contained 4 animals (total of 8 ovaries per group) and only 2 ovaries from each experimental group, randomized, are represented. The scale associated with the images is dimensioned in centimeters.

The results are expressed as ovarian mass in grams (g) and indicate that the recombinant forms of eCG (SEQ. ID. 17 and SEQ. ID. 19) exhibit in vivo bioactivity similar to native eCG (SEQ. ID. 5 and SEQ. ID. 7). These examples aid in the rationale for including hybrid forms of the hormone (composed of the alpha chain of equine and target animals) in this patent, since SEQ. ID. 17 and 19 show genetic similarity above 97% and structural similarities with the hybrid forms, which may be indicated for use in several species of mammals.

### Example 8 - Field tests for the evaluation of the activity of SEQ. ID. 17 in large animals (cattle):

The hormonal activity of SEQ. ID. 17 was evaluated in estrus synchronization protocols (IATF) in large mammals (Bovine) by Ultrasound (GE-Logiq and Transrectal transducer mod I-739, 8-12 Mhz) in females induced to estrus through hormonal protocols carried out by administration of eCG 300 IU and SEQ. ID. 17 30 µg.

The experimental model was based on Bos taurus indicus females, in homogeneous groups for the animal category (race, age, calving at least 1 time), with n = 127 for the eCG group and n = 50 for the SEQ ID. 17 group. The signs of estrus were verified by clinical evaluation and by comparison of follicular waves and ovulation (ultrasound).

The IATF protocol consisted of the introduction of the vaginal device for progesterone release (day 1), administration of eCG 300 IU and SEQ. ID. 17 30 µg and uterine evaluation by ultrasonography (day 8). Insemination was performed after the analysis by ultrasonography and follicular wave observation, where at least one follicle presented growth (1.4 mm/day) for each animal of both groups (day 10).

Figure 10 shows comparison of pregnancy rate which was performed 30 days post-insemination by trans-rectal ultrasonography in animals belonging to the inseminated groups from the IATF protocol using the eCG and SEQ. ID. 17. The results obtained showed a pregnancy rate of 50.23% for eCG, and of 48% for SEQ. ID. 17, where the national average pregnancy rate per IATF is 42%.

The analyzes showed the formation of cysts in 5.5% and twin formation in 1.59% in the eCG group, where the group SEQ. ID. 17 did not present the formation of cysts and twins.

### Applications

From an effective amount of the recombinant glycoprotein hormones of SEQ. ID. 17 and SEQ. ID. 19, for example, from 0.001 to 10,000 µg, together with pharmaceutically acceptable adjuvants, such as hormone scavengers or permeants and Nanotechnology-based Release Systems, it is possible to propose a pharmaceutical composition. These adjuvants aim to ensure pharmacokinetic and pharmacodynamic quality by ensuring the adequate bioactivity of these recombinant hormones in different animal reproduction protocols. Such composition is used for assisted animal reproduction comprising an effective amount of recombinant glycoprotein hormones. Such compositions will be for use in induction of ovulation; induction of superovulation; follicular growth; induction of estrus; reversal of anestrous; induction of puberty in animals of commercial interest or not, mammals in general, such as cattle, sheep, goats, pigs, horses, buffaloes, bison, antelopes, domestic and wild species of canines and felines, cetaceans, ursids and primates.

In addition, there is also the possibility of elaborating kits for use in induction of ovulation; induction of superovulation; follicular growth; induction of estrus; reversal of anestrous; induction of puberty; for use in IATF protocols (Fixed Time Artificial Insemination) FIC (In vitro Fertilization), TETF (Fixed Time Embryo Transfer) in animals of commercial interest or not.

Considering that chorionic gonadotrophins can be immunogenic (induce antibody production) and antigenic (recognized by antibodies) (Hervé et al. 2004, Forcada et al. 2011; Chopineau et al., 1993) it is possible to propose that the recombinant glycoprotein hormones in question can be used to obtain native (monoclonal and/or polyclonal) or recombinant (phage display) antibodies and that both these antibodies and recombinant glycoprotein hormones, and their derivatives (conjugates to enzymes, radiolabels and/or fluorochromes), may comprise kits for the detection of these two categories of molecules (hormones and anti-hormones) in biological samples or not.

Although the invention has been widely described, one person skilled in the art would find obvious that many changes and modifications may be made without covering said modifications by the scope of the invention.

### REFERENCES

Bousfield GR, Butnev VY, Butnev VY, Nguyen VT, Gray CM, Dias JA, MacColi R, Eisele L, Harvey Dj. Differential effects of alpha subunit Asparagine56 oligosaccharide structure on equine lutropin and follitropin hybrid conformation and receptor-binding activity. Biochemistry; 43(33):10817-10833, 2004.
Chopineau M, Maurel MC, Combarnous' Y, Durand P. Topography of equine chorionic gonadotropin epitopes relative to the luteinizing hormone and follicle-stimulating hormone receptor interaction sites. Mol Cell Endocrinol, 92(2):229-239, 1993.
Forcada F, Ait Amer-Meziane M, Abecia JA, Maurel MC, Cebriân-Pérez JA, Muiho-Blanco T, Asenjo B, Vazquez MI, Casao A. Repeated superovulation using a simplified FSH/eCG treatment for in vivo embryo production in sheep. Theriogenology, 75(4)769-776, 2011.
Galet C, Guillou F, Foulon-Gauze F, Combarnous Y, Chopineau M. The beta104-109 sequence is essential for the secretion of correctly folded single-chain beta alpha horse LH/CG and for its FSH activity. J Endocrinol, 203(1 ): 167-174, 2009.
Gopalkrishnan RV, Christiansen KA, Goldstein NI, DePinho RA, Fisher PB. Use of the human EF-1 alpha promoter for expression can significantly increase success in establishing stable cell lines with consistent expression: a study using the tetracycline-inducible system in human cancer cells. Nucleic Acids Res. Dec 15; 27(24): 4775-82. 1999.
Hervé V, Roy F, Bertin J, Guillou F, Maurel MC. Antiequine chorionicgonadotropin (eCG) antibodies generated in goats treated with eCG for the induction of ovulation modulate the luteinizing hormone and follicle-stimulating hormone bioactivities of eCG differently. Endocrinology, 145(1):294-303, 2004.
Min KS, Hattori N, Aikawa J, Shiota K, Ogawa T. Site-directed mutagenesis of recombinant equine chorionic gonadotropin/luteinizing hormone: differential role of oligosaccharides in luteinizing hormone- and follicle-stimulating hormone-iike activities. Endocr J 43(5):585-593, 1996. Min KS, Hiyama T, Seong HH, Hattori N, Tanaka S, Shiota K. Biological activities of tethered equine chorionic gonadotropin (eCG) and its deglycosylated mutants. J Reprod Dev, 50(3):297-304, 2004.
Moritz B, Becker PB, Göpfert U. CMV promoter mutants with a reduced propensity to productivity loss in CHO cells. Sei Rep. Nov 19; 5: 16952. doi: 10.1038/srep16952. 2015.
Moyle WR, Campbell RK, Myers RV, Bernard MP, Han Y, Wang X. Coevolution of ligand-receptor pairs. Nature, 368(6468): 251-255, 1994.
Mullis K, Faloona F, ScharfS, Saiki R, Horn G, Erlich H. Specific enzymatic amplification of DNA in vitro: the polymerase chain reaction. Cold Spring Harb Symp Quant Biol.;51 Pt 1: 263-73. 1986.
Murphy, BD. Equine chorionic gonadotropin: an enigmatic but essential tool. Anim Reprod, 9 (3): 223-230, 2012.
Sambrook, J.; Fritsch, E.F.; Maniatis, T: Molecular Cloning: A laboratory manual. 2nd ed. Cold Spring Harbor Laboratory Press, Cold Spring Harbor, N.Y. 1989.Sanger, F., Nicklen, S. And Coulson, AR. DNA sequencing with chain terminating inhibitors. Proc. Natl. Acad. Sci. USA, 74:5463-5467. 1977.

## Claims

1. A process for the production and purification of a hybrid chorionic gonadotropin, comprising the steps of:
(a) amplification and cloning a DNA molecule encoding a polypeptide selected from the group consisting of SEQ ID NO:23, SEQ ID NO: 25, SEQ ID NO: 27, SEQ ID NO: 29, SEQ ID NO:31, SEQ ID NO:33, SEQ ID NO:35 and SEQ ID NO:37 and the use of nucleotide sequences of primers of the different forms of chorionic gonadotrophin and of cleavage sites for restriction enzymes and of DNA sequences coding for a histidine tail and a proteolytic site for TEV-Tag protease;
(b) construction of the expression vectors comprising the sequences of step (a);
(c) transfection of a mammalian cell line with the vector of step (b), expression and analysis of cells;
(d) purification of the hybrid chorionic gonadotropin by affinity chromatography, wherein the hybrid chorionic gonadotropin is selected from the group consisting of SEQ ID NO: 23, SEQ ID NO: 25, SEQ ID NO: 27, SEQ ID NO: 29, SEQ ID NO: 31, SEQ ID NO: 33, SEQ ID NO: 35 and SEQ ID NO: 37.;
(e) dialysis and sterilization of the hybrid chorionic gonadotropin obtained in step (d).

2. The process of claim 1 wherein the DNA in step (a) is selected from the group consisting of SEQ ID NO: 22, SEQ ID NO: 24, SEQ ID NO: 26, SEQ ID NO: 28, SEQ ID NO: 30, SEQ ID NO: 32, SEQ ID NO: 34 and SEQ ID NO: 36.

3. A recombinant hybrid chorionic gonadotropin produced by the process of claims 1 or 2 for use in assisted animal reproduction technique in a mammal.

4. The recombinant hybrid chorionic gonadotropin for use according to claim 3, wherein the mammal is selected from the group consisting of cattle, sheep, goats, swine, horses, mules, bubalinps, bison, antelopes, domestic and wild species of canines and felines, cetaceans, ursids and primates.

5. The recombinant chorionic gonadotropin for use according to claim 4, wherein the mammal is cattle.

6. The recombinant chorionic gonadotropin for use according to claim 3, wherein the assisted animal reproduction technique is selected from the group consisting of: ovulation induction, induction of superovulation, follicular growth, induction of estrus, reversal of anestrous, puberty induction, use in FTAI protocols (Fixed-Time Artificial Insemination), IVF *(in vitro* fertilization) protocols and FTET protocols (Fixed Time Embryo Transfer).

7. The recombinant chorionic gonadotropin for use according to any of claims 3 to 6, wherein the recombinant chorionic gonadotropin is administered in an amount of 0.001 to 10,000 µg, observing the body weight of the target animal.

8. Expression vector of recombinant glycoprotein hormones selected from the group consisting of SEQ ID NO: 40, SEQ ID NO: 41, SEQ ID NO: 42, SEQ ID NO: 43, SEQ ID NO: 44, SEQ ID NO: 45, SEQ ID NO: 46 and SEQ ID NO: 47.

## Patentansprüche

1. Verfahren zur Herstellung und Reinigung eines hybriden Choriongonadotropins, umfassend die folgenden Schritte:
(a) Amplifikation und Klonierung eines DNA-Moleküls, das ein Polypeptid codiert, das aus der Gruppe bestehend aus SEQ ID NO: 23, SEQ ID NO: 25, SEQ ID NO: 27, SEQ ID NO: 29, SEQ ID NO: 31, SEQ ID NO: 33, SEQ ID NO: 35 und SEQ ID NO: 37 ausgewählt ist, und die Verwendung von Nukleotidsequenzen von Primern der unterschiedlichen Formen von Choriongonadotropin und von Spaltstellen für Restriktionsenzyme und von DNA-Sequenzen, die einen Histidinschwanz und eine proteolytische Stelle für TEV-Tag-Protease codieren;
(b) Konstruktion der Expressionsvektoren, die die Sequenzen aus Schritt (a) umfassen;
(c) Transfektion einer Säugetierzelllinie mit dem Vektor aus Schritt (b), Expression und Analyse der Zellen;
(d) Reinigung des hybriden Choriongonadotropins durch Affinitätschromatographie, wobei das hybride Choriongonadotropin aus der Gruppe bestehend aus SEQ ID NO: 23, SEQ ID NO: 25, SEQ ID NO: 27, SEQ ID NO: 29, SEQ ID NO: 31, SEQ ID NO: 33, SEQ ID NO: 35 und SEQ ID NO: 37 ausgewählt ist;
(e) Dialyse und Sterilisation des in Schritt (d) erhaltenen hybriden Choriongonadotropins.

2. Verfahren nach Anspruch 1, wobei die DNA in Schritt (a) aus der Gruppe bestehend aus SEQ ID NO: 22, SEQ ID NO: 24, SEQ ID NO: 26, SEQ ID NO: 28, SEQ ID NO: 30, SEQ ID NO: 32, SEQ ID NO: 34 und SEQ ID NO: 36 ausgewählt ist.

3. Rekombinantes hybrides Choriongonadotropin, hergestellt nach dem Verfahren nach Anspruch 1 oder 2, zur Verwendung in der assistierten Reproduktionstechnik bei Säugetieren.

4. Rekombinantes hybrides Choriongonadotropin zur Verwendung nach Anspruch 3, wobei das Säugetier ausgewählt ist aus der Gruppe bestehend aus Rindern, Schafen, Ziegen, Schweinen, Pferden, Maultieren, Büffeln, Bisons, Antilopen, domestizierten und wilden Arten von Hunden und Katzen, Cetaceen, Ursidae und Primaten.

5. Rekombinantes Choriongonadotropin zur Verwendung nach Anspruch 4, wobei das Säugetier Rinder sind.

6. Rekombinantes Choriongonadotropin zur Verwendung nach Anspruch 3, wobei die Technik zur assistierten Reproduktion von Tieren ausgewählt ist aus der Gruppe bestehend aus: Ovulationsinduktion, Induktion von Superovulation, Follikelwachstum, Induktion von Östrus, Umkehrung von Anöstrus, Pubertätsinduktion, Verwendung in FTAI-Protokollen (zeitlich festgelegte künstliche Besamung), IVF-Protokollen (In-vitro-Fertilisation) und FTET-Protokollen (zeitlich festgelegter Embryotransfer).

7. Rekombinantes Choriongonadotropin zur Verwendung nach einem der Ansprüche 3 bis 6, wobei das rekombinante Choriongonadotropin unter Berücksichtigung des Körpergewichts des Zieltieres in einer Menge von 0,001 bis 10.000 µg verabreicht wird.

8. Expressionsvektor für rekombinante Glykoproteinhormone, ausgewählt aus der Gruppe bestehend aus SEQ ID NO: 40, SEQ ID NO: 41, SEQ ID NO: 42, SEQ ID NO: 43, SEQ ID NO: 44, SEQ ID NO: 45, SEQ ID NO: 46 und SEQ ID NO: 47.

## Revendications

1. Procédé de production et de purification d'une gonadotrophine chorionique hybride, comprenant les étapes de :
(a) amplification et clonage d'une molécule d'ADN codant pour un polypeptide sélectionné dans le groupe constitué par SEQ ID NO : 23, SEQ ID NO : 25, SEQ ID NO : 27, SEQ ID NO : 29, SEQ ID NO : 31, SEQ ID NO : 33, SEQ ID NO: 35 et SEQ ID NO: 37 et l'utilisation de séquences nucléotidiques d'amorces des différentes formes de gonadotrophine chorionique et de sites de clivage pour des enzymes de restriction et de séquences d'ADN codant pour une queue d'histidine et un site protéolytique pour la protéase TEV-Tag ;
(b) construction des vecteurs d'expression comprenant les séquences de l'étape (a) ;
(c) transfection d'une lignée cellulaire de mammifère avec le vecteur de l'étape (b), expression et analyse de cellules ;
(d) purification de la gonadotrophine chorionique hybride par chromatographie d'affinité, dans lequel la gonadotrophine chorionique hybride est sélectionnée dans le groupe constitué par SEQ ID NO : 23, SEQ ID NO : 25, SEQ ID NO : 27, SEQ ID NO : 29, SEQ ID NO : 31, SEQ ID NO : 33, SEQ ID NO : 35 et SEQ ID NO : 37 ;
(e) dialyse et stérilisation de la gonadotrophine chorionique hybride obtenue dans l'étape (d).

2. Procédé selon la revendication 1, dans lequel l'ADN dans l'étape (a) est sélectionnée dans le groupe constitué par SEQ ID NO : 22, SEQ ID NO : 24, SEQ ID NO : 26, SEQ ID NO : 28, SEQ ID NO : 30, SEQ ID NO : 32, SEQ ID NO : 34 et SEQ ID NO : 36.

3. Gonadotrophine chorionique hybride recombinante produite par le procédé selon les revendications 1 ou 2, pour son utilisation dans une technique de reproduction assistée des animaux chez un mammifère.

4. Gonadotrophine chorionique hybride recombinante pour son utilisation selon la revendication 3, dans laquelle le mammifère est sélectionné dans le groupe constitué par les bovins, les moutons, les chèvres, les porcins, les chevaux, les mules, les bubalins, le bison, les antilopes, les espèces domestiques et sauvages des canines et des félines, les cétacés, les ursidés et les primates.

5. Gonadotrophine chorionique recombinante pour son utilisation selon la revendication 4, dans laquelle le mammifère est du bovin.

6. Gonadotrophine chorionique recombinante pour son utilisation selon la revendication 3, dans laquelle la technique de reproduction assistée des animaux est sélectionnée dans le groupe constitué par : l'induction de l'ovulation, l'induction de la surovulation, la croissance folliculaire, l'induction de l'oestrus, l'inversion de l'anoestrus, l'induction de la puberté, l'utilisation dans des protocoles de IATF (insémination artificielle à temps fixe), les protocoles de FIV (fécondation *in vitro*) et les protocoles de TETF (transfert embryonnaire à temps fixe).

7. Gonadotrophine chorionique recombinante pour son utilisation selon l'une quelconque des revendications 3 à 6, dans laquelle la gonadotrophine chorionique recombinante est administrée en une quantité comprise entre 0,001 et 10.000 µg, en observant le poids corporel de l'animal cible.

8. Vecteur d'expression d'hormones glycoprotéiques recombinantes sélectionnées dans le groupe constitué par SEQ ID NO : 40, SEQ ID NO : 41, SEQ ID NO : 42, SEQ ID NO : 43, SEQ ID NO : 44, SEQ ID NO : 45, SEQ ID NO : 46 et SEQ ID NO : 47.
